# EUROPEAN PATENT APPLICATION

(11) **EP 4 732 800 A1**
(43) Date of publication of application: **29.04.2026**
(21) Application number: 24842377.4
(22) Date of filing: 16.07.2024
(51) Int. Cl.: A61B 18/14, A61B 18/12

(54) **SURGICAL INSTRUMENT AND SURGICAL SYSTEM**

(30) Priority: 17.07.2023 CN 202310879522
(71) Applicant: Wuhan Mindray Scientific Co., Ltd., Hubei 430206 (CN); Shenzhen Mindray Bio-Medical Electronics Co., Ltd., Shenzhen, Guangdong 518057 (CN)
(72) Inventor: WANG, Kui Wen, Shenzhen, Guangdong 518057 (CN); HUANG, Xu, Shenzhen, Guangdong 518057 (CN); GENG, Ping, Shenzhen, Guangdong 518057 (CN)
(74) Representative: KIPA AB
(86) International application number: PCT/CN2024/105795
(87) International publication number: WO 2025/016388

(57) **Abstract**

A surgical instrument and a surgical system. The surgical instrument comprises a handle assembly, a jaw assembly, and a tube assembly. The tube assembly comprises a pull rod and a tube. A second fluid channel is formed between the outer peripheral wall of the pull rod and the inner peripheral wall of the tube. An opening communicated with the second fluid channel is formed at the position on the tube close to the jaw assembly. A first fluid channel is communicated with the second fluid channel and is used for being connected to a fluid delivery device so as to achieve a fluid path between the opening and the fluid delivery device.

## Description

This disclosure claims priority to a Chinese patent application filed to China Patent Office on July 17, 2023, with an application number 202310879522.2, and titled "Surgical Instrument and Surgical System", the entire contents of which are incorporated by reference into this disclosure.

### TECHNICAL FIELD

The disclosure relates to a technical field of medical devices, and more particularly to a surgical instrument and a surgical system.

### BACKGROUND

At present, minimally invasive surgery occupies an important position in surgical operations, and surgical instruments are commonly used instruments in minimally invasive surgery. In clinical surgical operations, in addition to cutting or electrocoagulation of biological tissues, another instrument is also needed to for cooperating another operation. For example, during a laparoscopic surgery, a smoke generated by a surgical instrument will affect a surgical field of view and interfere with an operation of a doctor. Traditional smoke removal methods usually require a use of a puncture device or a flushing tube. While removing a smoke, a surgical operation needs to be interrupted and a new instrument need to be introduced, thereby reducing a surgical efficiency.

### SUMMARY

In view of this, this disclosure proposes a surgical instrument and a surgical system.

A first aspect of this disclosure provides a surgical instrument including:
a handle assembly, which includes a handle for holding and a trigger capable of moving relative to the handle, wherein the handle is provided with a first fluid channel;
a jaw head assembly, which includes a first jaw head and a second jaw head, wherein the first jaw head and the second jaw head are capable of pivoting relative to each other , so as to open or close the jaw head assembly, wherein at least one of the first jaw head and the second jaw head is configured to implement a treatment on a biological tissue;
a tubing assembly, which includes a pull rod and a tube which is sleeved outside the pull rod, wherein the tubing assembly is configured to connect the jaw head assembly and the handle; when the trigger moves relative to the handle, the jaw head assembly moves under an action of the pull rod, which pull rod is in a kinematic fit with the trigger, so as to enable the first jaw head and the second jaw head to pivot relative to each other;
wherein, a second fluid channel is formed between an outer peripheral wall of the pull rod and an inner peripheral wall of the tube; a position of the tube, which position is adjacent to the jaw head assembly, is provided with an opening which is connected with the second fluid channel; wherein the first fluid channel is connected with the second fluid channel and is further connected with a fluid delivery device, so as to form a fluid path between the opening and the fluid delivery device.

A second aspect of this disclosure provides a surgical system, including a surgical energy host and the above-mentioned surgical instrument, wherein the surgical energy host is connected with the surgical instrument to provide energy to the surgical instrument.

It can be seen from the above technical solution that a surgical instrument proposed in this disclosure is capable of discharging a fluid, such as a smoke generated during a surgical process, by providing an opening at a position on a tube, which position is adjacent to a jaw head assembly, and implementing a fluid path between the opening and a fluid delivery device through arranging a first fluid channel and a second fluid channel inside the surgical instrument. This disclosure can implement a tissue treatment function and a fluid delivery function through one surgical instrument, thus reducing an instrument change during a surgery, eliminating a requirement of introducing a new instrument, such that a surgical operation does not need to be interrupted and no new instrument needs to be introduced, thereby improving a surgical efficiency, shortening surgery time, and reducing a workload of a doctor.

### BRIEF DESCRIPTION OF THE DRAWINGS

In order to provide a clearer explanation for embodiments of this disclosure or technical solutions in the prior art, a brief introduction is given to accompanying drawings required for description of the embodiments or the prior art. It is obvious that the accompanying drawings described below are only embodiments of this disclosure. For those skilled in the art, other drawings can be obtained based on the provided drawings without creative labour.
FIG. 1 is a structural diagram for a surgical instrument according to an embodiment of this disclosure.
FIG. 2 is a structural diagram for a handle assembly according to an embodiment of this disclosure.
FIG. 3 is a cross-sectional diagram for a structure shown in FIG. 2.
FIG. 4 is a cross-sectional diagram for a tubing assembly adjacent to a jaw head assembly according to an embodiment of this disclosure.
FIG. 5 is a cross-sectional diagram for a connection point between a junction structure and a second fluid channel in a horizontal direction of FIG. 1.
FIG. 6 is a cross-sectional diagram or a connection point between a junction structure and a second fluid channel in a vertical direction of FIG. 1.
FIG. 7 is a partially enlarged diagram for a structure shown in FIG. 6.
FIG. 8 is a structural diagram for a jaw head assembly of a surgical instrument according to an embodiment of this disclosure, when the jaw head assembly is opened, showing a cross-sectional view of a handle.
FIG. 9 is a structural diagram for a jaw head assembly of a surgical instrument according to an embodiment of this disclosure, when the jaw head assembly is closed, showing a cross-sectional view of a handle.
FIG. 10 is a cross-sectional diagram for a handle of a surgical instrument according to an embodiment of this disclosure, showing a variation of a switch structure.
FIG. 11 is a structural diagram for a switch structure in a first state according to an embodiment of this disclosure.
FIG. 12 is a structural diagram for a switch structure in a second state according to an embodiment of this disclosure.
FIG. 13 is a structural diagram for a handle of a surgical instrument according to an embodiment of this disclosure, showing another variation of a switch structure.
FIG. 14 is a structural diagram for a switch structure in a first state, in a structure shown in FIG. 13.
FIG. 15 is a structural diagram for a switch structure in a second state, in a structure shown in FIG. 13.
FIG. 16 is a diagram for an internal structure of a switch structure in a structure shown in FIG. 13.

### Description of reference numbers:

100, surgical instrument; 10, handle assembly; 11, handle; 11a, upper side; 11b, lower side; 11c, front side; 11d, rear side; 111, main body; 1111, head portion; 1112, tail portion; 112, holding portion; 113, second position-limiting flange; 12, trigger; 121, connection portion; 122, operation portion; 1221, inner hole; 123, rotation shaft; 13, rotation wheel; 131, extension portion; 132, first position-limiting ring-groove; 133, first position-limiting flange; 134, second position-limiting ring-groove; 20, jaw head assembly; 21, first jaw head; 22, second jaw head; 30, tubing assembly; 31, tube; 31a, first insulation tube; 311, opening; 312, insulation layer; 313, front section; 32, pull rod; 32a, second insulation tube; 33. second fluid channel; 34, connection seat; 40, junction structure; 41, inner cavity; 42, first interface; 421, first end; 422, second end; 43, second interface; 44, first sealing ring; 45, sealing member; 45a, second sealing ring; 46, position-limiting member; 461, blocking portion; 462, covering portion; 47, abutting portion; 50, first fluid channel; 51, first section; 52, second section; 60, switch structure; 61, valve body; 611, reset member; 612, connection channel; 613, first outlet; 614, second outlet; 62, switch member; 63, valve; 631, through hole; 70, function button.

### DESCRIPTION OF THE PREFERRED EMBODIMENTS

Technical solutions in embodiments of this disclosure will be clearly and completely described in conjunction with the accompanying drawings in the followings. Obviously, the described embodiments are only a portion of the embodiments of this disclosure, not all of them. Based on the embodiments of this disclosure, all other embodiments obtained by ordinary skilled persons in the art without creative labor are within a scope of protection of this disclosure.

It should also be understood that, terms used in the description of this disclosure are only used to describe specific embodiments and are not intended to limit this disclosure. As used in the description and the appended claims, the singular forms "a," "an," and "the" are intended to include the plural forms as well, unless the context clearly indicates otherwise.

It should be further understood that, a term "and/or" used in the description and the appended claims refers to and includes any and all possible combinations of one or more of the associated listed items.

It should be noted that, all directional indications in embodiments of this disclosure (such as up, down, left, right, front, back, etc.) are only used to explain a relative position relationship, movement state, etc. between various components in a certain specific posture. If the specific posture changes, the directional indication will also change accordingly.

At present, minimally invasive surgery occupies an important position in surgical operations, and surgical instruments are commonly used instruments in minimally invasive surgery. In clinical surgical operations, in addition to cutting or electrocoagulation of biological tissues, another instrument is also needed to for cooperating another operation. For example, during a laparoscopic surgery, an energy instrument, such as a bipolar electrocoagulation instrument, an ultrasonic scalpel and other surgical instruments, will inevitably produce a smoke during use. The smoke produced by the surgical instrument will affect a surgical field of view and interfere with an operation of a doctor. Traditional smoke removal methods usually require a use of a puncture device or a flushing tube. While removing a smoke, a surgical operation needs to be interrupted and a new instrument need to be introduced, thereby reducing a surgical efficiency.

Therefore, this disclosure provides a surgical instrument, inside which a first fluid channel and a second fluid channel are arranged for delivering a fluid, so as to reduce an instrument change during surgery, improve a surgical efficiency, shorten surgery time, and reduce workload of a doctor.

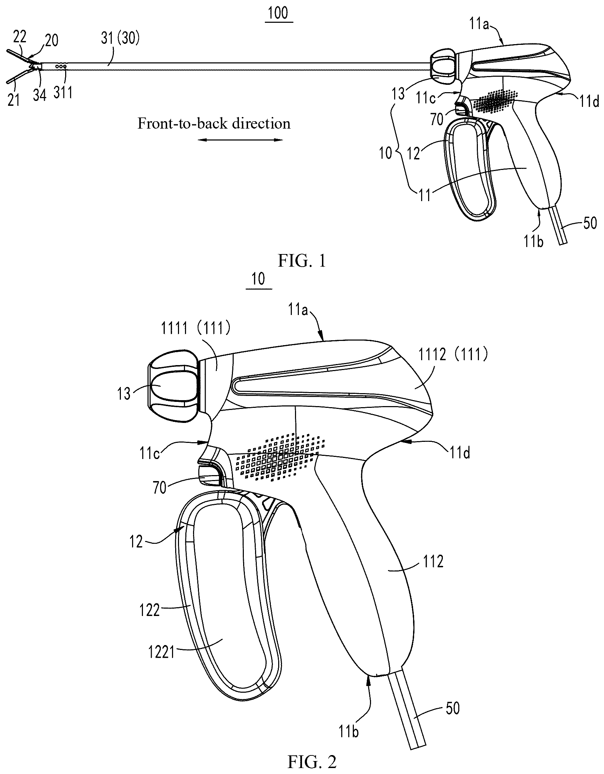

Referring to FIGS. 1-4, an embodiment of this disclosure provides a surgical instrument 100, including a handle assembly 10, a jaw head assembly 20 and a tubing assembly 30. The handle assembly 10 includes a handle 11 for holding and a trigger 12 capable of moving relative to the handle 11. The handle 11 is provided with a first fluid channel 50. The jaw head assembly 20 includes a first jaw head 21 and a second jaw head 22. The first jaw head 21 and the second jaw head 22 are capable of pivoting relative to each other, so as to open or close the jaw head assembly 20. At least one of the first jaw head 21 and the second jaw head 22 is configured to implement a treatment on a biological tissue. Exemplarily, the surgical instrument 100 proposed in an embodiment of this disclosure can be a surgical instrument, such as a bipolar coagulation instrument, a monopolar coagulation instrument with double jaw heads, an ultrasonic scalpel, a surgical stapling instrument, etc. The treatment on the biological tissue can be a treatment in which energy is applied to the biological tissue, such as cutting by ultrasonic vibration or coagulation treatment by bipolar or monopolar coagulation, etc., or can be a treatment in which no energy is applied to the biological tissue, such as a stitching treatment by a surgical stapling instrument, etc.

The tubing assembly 30 includes a pull rod 32 and a tube 31 sleeved outside the pull rod 32, wherein the tubing assembly 30 is configured to connect the jaw head assembly 20 and the handle 11. A kinematic fit exists between the pull rod 32 and the trigger 12. When the trigger 12 moves relative to the handle 11, the jaw head assembly 20 moves under an action of the pull rod 32, which pull rod 32 is in a kinematic fit with the trigger 12, so as to enable the first jaw head 21 and the second jaw head 22 to pivot relative to each other, in order to open or close the jaw head assembly 20. When operating the surgical instrument 100, an operator can hold the handle 11 and extend the jaw head assembly 20 into a lesion site inside a human body through a slender tubing assembly 30. Then, the trigger 12 is operated to move relative to the handle 11 to drive the jaw head assembly 20 to clamp a biological tissue inside a preset region, so as to enable the jaw head assembly 20 to implement a treatment on the biological tissue in a preset region without affecting other tissue regions.

Wherein, a second fluid channel 33 is formed between an outer peripheral wall of the pull rod 32 and an inner peripheral wall of the tube 31. A position of the tube 31, which position is adjacent to the jaw head assembly 20, is provided with an opening 311, which opening 311 is connected with the second fluid channel 33. The first fluid channel 50 is connected with the second fluid channel 33 and is further connected with a fluid delivery device, so as to form a fluid path between the opening 311 and the fluid delivery device.

The surgical instrument 100 proposed in an embodiment of this disclosure can discharge a fluid, such as a smoke generated during a surgical process, by providing an opening 311 at a position on the tube 31, which position is adjacent to the jaw head assembly 20, and implementing a fluid path between the opening 311 and the fluid delivery device through arranging a first fluid channel 50 and a second fluid channel 33 inside the surgical instrument. This disclosure can implement a tissue treatment function and a fluid delivery function through one surgical instrument, thus reducing an instrument change during a surgery, eliminating a requirement of introducing a new instrument, such that a surgical operation does not need to be interrupted and no new instrument needs to be introduced, thereby improving a surgical efficiency, shortening surgery time, and reducing a workload of a doctor.

Optionally, the fluid delivery device may be at least one of a negative pressure source and a positive pressure source. For example, the negative pressure source may be a fan, or another negative pressure device capable of generating a negative pressure airflow to discharge a fluid. The positive pressure source may be an input pump, or another power device capable of generating a driving force to deliver a fluid.

In some usage scenarios, the fluid delivery device includes a first negative pressure source, and the first fluid channel 50 is connected with the first negative pressure source, so as to discharge, through the opening 311, a smoke, which is generated when the jaw head assembly 20 applies energy to a biological tissue. At this time, the fluid path between the opening 311 and the fluid delivery device can be used to exhaust gas, so as to prevent a smoke generated during a surgery from affecting a field of view of a doctor.

In other usage scenarios, the fluid delivery device may include a positive pressure source, and the first fluid channel may also be connected with the positive pressure source, so as to drive a cleaning fluid to flow out of the opening 311 to the jaw head assembly 20, so as to clean a biological tissue. Optionally, the fluid delivery device may include a second negative pressure source, wherein the first fluid channel 50 is connected with the second negative pressure source, so as to discharge a waste fluid from the jaw head assembly 20 through the opening 311. At this time, the fluid path between the opening 311 and the fluid delivery device can be used to input and/or output a liquid.

Exemplarily, the surgical instrument 100 can be a bipolar coagulation forceps, wherein the first jaw head 21 and the second jaw head 22 are both forceps jaws, the first jaw head 21 is connected with a positive pole of a power supply that supplies energy to the surgical instrument 100, and the second jaw head 22 is connected with a negative pole of the power supply that supplies energy to the surgical instrument 100. The first jaw head 21 and the second jaw head 22 are respectively positive and negative poles for applying electrical energy to a biological tissue for electrocoagulation treatment.

Of course, the surgical instrument can also be a monopolar coagulation forceps, the first jaw head 21 and the second jaw head 22 are both forceps jaws, and one of the first jaw head 21 and the second jaw head 22 is connected with a pole of a power supply that supplies energy to the surgical instrument.

Of course, the surgical instrument can also be an ultrasonic scalpel, in which one of the first jaw head 21 and the second jaw head 22 is a jaw, and the other of the first jaw head 21 and the second jaw head 22 is an ultrasonic scalpel head, which applies an ultrasonic vibration to a biological tissue, so as to perform an operation, such as cutting or coagulation.

Of course, the surgical instrument can also be a surgical stapling instrument, in which the first jaw head 21 is a mounting seat of the surgical stapling instrument, on which mounting seat stitching staples are mounted, and the second jaw head 22 is a cutting knife of the surgical stapling instrument. The surgical stapling instrument can remove a biological tissue through the cutting knife and stitch the biological tissue through the stitching staples at the same time.

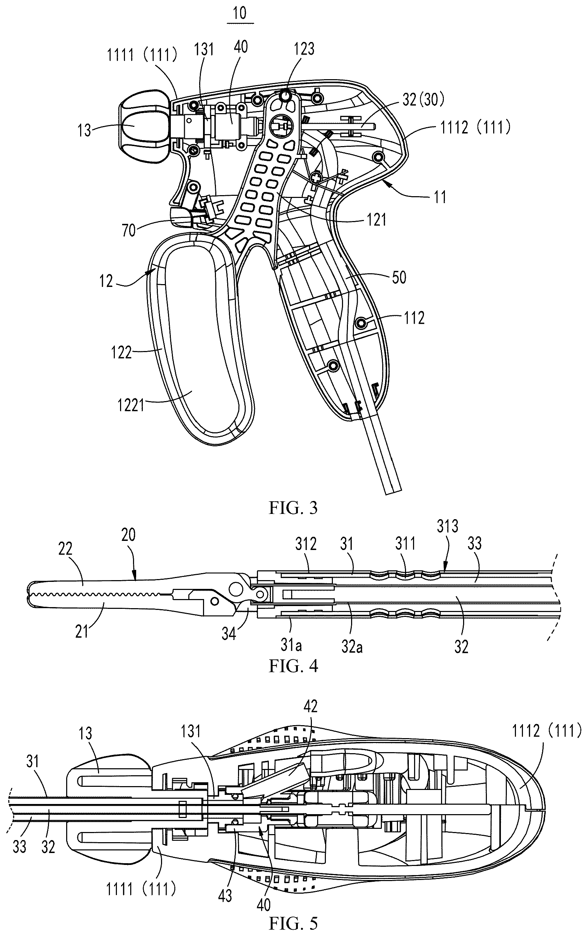

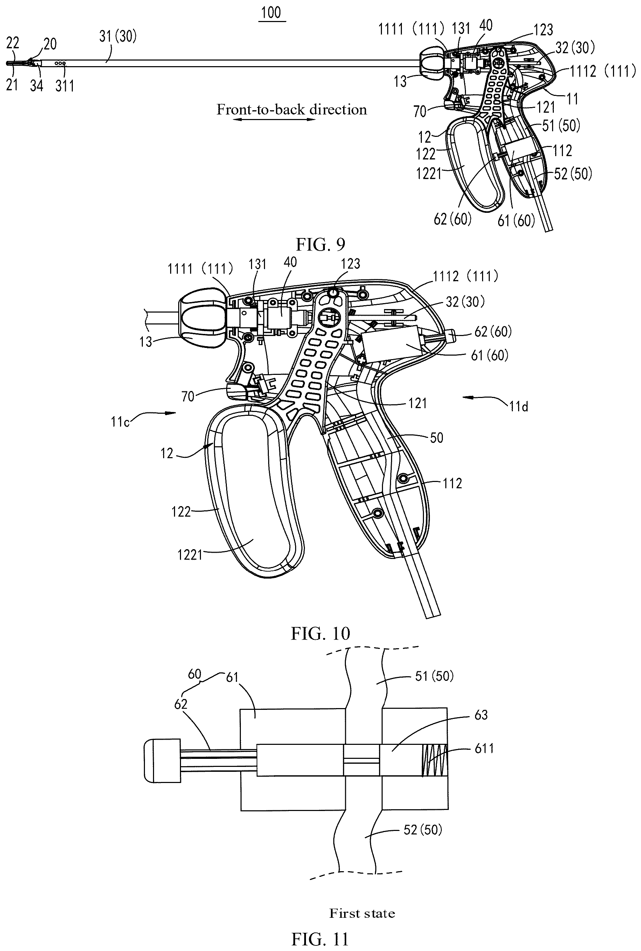

In some embodiments, as shown in FIG. 1 and FIG. 4, the pull rod 32 is hinged with the second jaw head 22, and the second jaw head 22 is hinged with the first jaw head 21 through an insulation member, so as to enable the second jaw head 22 to rotate relative to the first jaw head 21. In this embodiment, the tube 31 can be connected with the first jaw head 21 through a connection seat 34. When the pull rod 32 moves along an axial direction, the pull rod 32 is capable of driving the second jaw head 22 to rotate relative to the first jaw head 21, so as to open or close the jaw head assembly 20. As shown in FIG. 9, taking a doctor controlling to close the jaw head assembly 20 as an example, when the doctor operates the trigger 12, the trigger 12 drives the pull rod 32 to move inside the tube 31 toward the handle 11, and the moved pull rod 32 pulls the second jaw head 22 which is hinged therein, so as to enable the first jaw head 21 and the second jaw head 22 to rotate towards each other for closing.

Optionally, the connection seat 34 can be integrally formed with the tube 31, or be separately formed but connected with the tube 31. For example, only one of the first jaw head 21 and the second jaw head 22 may be rotatable relative to the other, or both of the first jaw head 21 and the second jaw head 22 may be rotatable relative to the other, so as to open or close the jaw head assembly 20.

As an embodiment, the first jaw head 21 is hinged with the connection seat 34, and the first jaw head 21 is configured to rotate synchronously when the second jaw head 22 rotates, so as to cooperate with the second jaw head 22 to rotate to open or close the jaw head assembly 20. In this embodiment, the first jaw head 21 can rotate synchronously with the first jaw head 22 to clamp a biological tissue.

Of course, as another embodiment, the first jaw head 21 and the connection seat 34 can be fixedly connected. At this time, the second jaw head 22 can rotate relative to the first jaw head 21, so as to open or close the jaw head assembly 20 to clamp a biological tissue.

In some embodiments, as shown in FIG. 4, the tube 31 is provided with an insulation layer 312, which at least is affixed at the opening 311 to prevent an electric leakage at the opening 311, while using the surgical instrument 100, thereby strengthening insulation, avoiding a breakdown risk, and ensuring an electrical safety. In this embodiment, the insulation layer 312 may be a coating applied on a surface of the tube 31, or may be silicone or another insulation material injection-molded on a surface of the tube 31, thereby preventing the tube 31 from leaking electricity at the opening 311.

In some embodiments, the tube 31 is in electrical connection with the first jaw head 21, and the pull rod 32 is in electrical connection with the second jaw head 22, so as to enable at least one of the first jaw head 21 and the second jaw head 22 to apply energy from a surgical energy host to a biological tissue. The tube 31 and the pull rod 32 are relatively insulated from each other to ensure electrical safety. Optionally, an outer surface of the tube 31 is provided with a first insulation structure, and an outer surface of the pull rod 32 is provided with a second insulation structure, so as to achieve relative insulation between the tube 31 and the pull rod 32.

As an embodiment, as shown in FIG. 4, the first insulation structure can be a first insulation tube 31a sleeved outside the tube 31, and the first jaw head 21 is connected with a positive pole of a power supply that supplies energy to the surgical instrument 100 through the tube 31. The second insulation structure can be a second insulation tube 32a sleeved outside the pull rod 32, and the second jaw head 22 is connected with a negative pole of the power supply that supplies energy to the surgical instrument 100 through the pull rod 32, so as to enable the first jaw head 21 and the second jaw head 22 to apply energy from a surgical energy host to a biological tissue. The first insulation structure and the second insulation structure are provided to achieve insulation between the tube 31 and the pull rod 32.

Specifically, since an opening 311 is provided on the tube 31, a hole also needs to be punched at a corresponding position of the first insulation tube 31a. A surgical instrument, such as a bipolar coagulation instrument and an ultrasonic scalpel, has a compact structure and is prone to breakdown. A conductive body fluid is easily introduced during a fluid delivery, thus causing insulation failure and instrument failure. Therefore, in order to strengthen insulation, the tube 31 can be provided with an insulation layer 312, which is at least affixed at the opening 311, thereby avoiding a breakdown risk and ensuring an electrical safety.

Of course, if an entire outer peripheral wall of the tube 31 is affixed with an insulation layer 312, there is no need to install the first insulation tube 31a. It can be understood that the second insulation structure outside the pull rod 32 can also be an insulation coating applied on a surface of the tube 31, or silicone or another insulation material injection-molded on a surface of the pull rod 32.

In some embodiments, an affixing region of the insulation layer 312 on the tube 31 extends from an outer peripheral wall at the opening 311 to an inner peripheral wall of the opening 311 through the opening 311, so as to prevent an electrical leakage at the opening 311, thereby strengthening insulation and ensuring an electrical safety.

In some embodiments, as shown in FIG. 4, the tube 31 includes a front section 313, which is adjacent to the jaw head assembly 20, wherein the opening 311 is provided at the front section 313. An affixing region of the insulation layer 312 extends from an outer peripheral wall of the front section 313 to an inner peripheral wall of the front section 313 through the opening 311, so as to strengthen insulation.

Optionally, the insulation layer 312 may be affixed to the tube 31 at a position which is just adjacent to the opening 311, or the insulation layer 312 may be affixed to a section region of the tube 31, which section region is adjacent to the jaw head assembly 20 and provided with the opening 311, that is, the outer peripheral wall of the front section 313 and the inner peripheral wall of the front section 313, which inner peripheral wall is adjacent to the opening 311, are both affixed with the insulation layer 312. During processing, the front section 313 of the tube 31 can be placed into a spraying region to facilitate processing.

In some embodiments, as shown in FIG. 1 and FIG. 4, a plurality of openings 311 are provided on the tube 31, and the plurality of openings 311 are arranged in an array on the tube 31. In this embodiment, a delivery efficiency of fluid can be improved by arranging a plurality of openings 311 in an array.

Optionally, the plurality of openings 311 may be arranged in a linear shape, in a rectangular shape, or in a diamond-shape, along the axial direction of the tube 31, or the plurality of openings 311 may be arranged in an array around the tube 31. The plurality of openings 311 are arranged in a compact structure, so as to centrally guide a fluid to the second fluid channel 33, thereby improving a delivery efficiency of fluid.

Of course, in other embodiments, the plurality of openings 311 may also be arranged obliquely, or in other arrangements.

In some embodiments, shapes of the plurality of openings 311 on the tube 31 may be the same or different. A shape of an opening 311 can be circular, square, diamond-shaped, or irregular-shaped.

In some embodiments, sizes of the plurality of openings 311 on the tube 31 may be the same or different.

In some embodiments, in an axial direction of the tube 31, the insulation layer 312 may cover a length of 2% to 20% of an axial length of the tube 31. In this embodiment, if a coverage area of the insulation layer 312 is too large, the cost will increase. If the affixing region is too small, it will be difficult to cover a sufficient range. Therefore, the coverage area of the insulation layer 312 is set within the above range, so as to ensure to cover a sufficient range and improve an electrical safety performance, while controlling the coverage area to save a processing cost.

Exemplarily, a length of the tube 31, which length is covered by the insulation layer 312, can be 2%, 3%, 4%, 5%, 6%, 7%, 8%, 9%, 10%, 11%, 12%, 13%, 14%, 15%, 16%, 17%, 18%, 19% or 20% of an axial length of the tube 31.

In some embodiments, the insulation layer 312 may be a coating formed by thin-film deposition. In this embodiment, thin-film deposition can be used to form the insulation layer 312. The thin-film deposition can ionize substances under vacuum and then combine positive and negative ions to form chemical bonds with a strong binding force, wherein the ionization forms atomic particles which are impacted onto the tube 31 through an electric field, and such atomic particles can be deposited densely, so as to generate a relatively strong binding force.

Exemplarily, thin-film deposition includes physical vapor deposition (PVD) and chemical vapor deposition (CVD). A main method of physical vapor deposition includes vacuum evaporation, sputtering coating, arc plasma coating, ion plating and molecular beam epitaxy. Chemical vapor deposition is a process that uses gaseous substances to produce chemical reactions and transport reactions on solids and produce solid deposits.

In other embodiments, the insulation layer 312 may also be a coating formed by spraying. In this embodiment, a spraying process includes thermal spraying and cold spraying. Thermal spraying refers to heating and melting a coating material, atomizing the coating material into extremely fine particles with a high-speed airflow, and spraying the coating material onto a workpiece surface at a very high speed, so as to form a coating. Cold spraying refers to spraying coating powder onto a substrate by supersonic gas and solid two-phase airflow, at a room temperature or a lower temperature, so as to form a dense coating.

For example, when processing the insulation layer 312, a spraying region or a coating region can be limited to the front section 313 of the tube 31, wherein the opening 311 is provided at which front section 313. A paint or a coating material will enter an interior of the tube 31 from the opening 311, so as to enable all of the outer peripheral wall of the front section 313 of the tube 31, the inner wall of the opening 311, and the inner peripheral wall of the front section 313 which inner peripheral wall is adjacent to the opening 311, to be affixed with the insulation layer 312, so as to strengthen insulation.

Of course, in other embodiments, the insulation layer 312 may also be formed by other processes, such as coating, dipping, spraying, brushing, drying, melting, laser curing, UV curing, anodizing, electroplating, thick film high-velocity oxygen flame plasma, and other suitable material application techniques. The processing technology used can form an insulation layer 312 on the surface of the tube 31 to strengthen insulation. The coating can be completed in one or more applications.

In some embodiments, a thickness of the insulation layer 312 is 1 µm-200 µm. When the insulation layer 312 is a coating formed by thin-film deposition, the thickness of the insulation layer 312 is 1 µm-10 µm. Exemplarily, the thickness of the insulation layer 312 may be 1 µm, 2 µm, 3 µm, 4 µm, 5 µm, 6 µm, 7 µm, 8 µm, 9 µm, or 10 µm. When the insulation layer 312 is a coating formed by a spraying process, the thickness of the insulation layer 312 is 10 µm-200 µm. Exemplarily, the thickness of the insulation layer 312 may be 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm or 200 µm.

In some embodiments, a material of the insulation layer 312 includes, but is not limited to, at least one of polytetrafluoroethylene (PTFE) and diamond-like carbon (DLC). In this embodiment, the insulation layer 312 made of polytetrafluoroethylene, has characteristics of corrosion resistance and wear resistance; the insulation layer 312 made of diamond-like carbon, has excellent properties of both diamond and graphite, and has high hardness, high resistivity, good optical property and excellent tribological property.

It should be noted that, the surgical instrument 100 of this disclosure is not limited to use the tube 31 and the pull rod 32 for electrical conduction. For example, in other embodiments, the tube 31 and the pull rod 32 are insulated, and the surgical instrument 100 may also include a first wire and a second wire, the first wire is in electrical connection with the first jaw head 21, and the second wire is in electrical connection with the second jaw head 22, so as to enable at least one of the first jaw head 21 and the second jaw head 22 to be capable of applying energy from a surgical energy host to a biological tissue.

Exemplarily, the first wire and the second wire can be routed inside the second fluid channel 33, so as to rationally utilize space and reduce a volume of the surgical instrument 100.

For example, in other embodiments, the pull rod 32 includes a first conductive unit and a second conductive unit that are independent of each other, wherein the first conductive unit is in electrical connection with the first jaw head 21, and the second conductive unit is in electrical connection with the second jaw head 22, so as to enable at least one of the first jaw head 21 and the second jaw head 22 to be capable of applying energy from a surgical energy host to a biological tissue.

In some embodiments, as shown in FIG. 3 and FIG. 5-FIG. 7, the surgical instrument 100 further includes a junction structure 40, which is configured to seal and connect the first fluid channel 50 and the second fluid channel 33, so as to from a fluid path between the opening 311 and the fluid delivery device. In this embodiment, providing the junction structure facilitates a connection between the first fluid channel 50 and the second fluid channel 33 for achieving fluid delivery.

In some embodiments, as shown in FIGS. 5-7, the junction structure 40 includes an inner cavity 41, and a first interface 42 and a second interface 43 which are connected with the inner cavity 41. The first interface 42 is configured to seal and connect the inner cavity 41 and the first fluid channel 50, the second interface 43 is configured to seal and connect the inner cavity 41 and the second fluid channel 33. In this embodiment, the above-mentioned arrangement guides a fluid, which enters into the second fluid channel 33, to flow into the inner cavity 41 through the second interface 43, and further flow into the first fluid channel 50 through the first interface 42, and then be discharged through a negative pressure source, or guides a fluid outputted through a positive pressure source to be inputted through the opening 311, so as to prevent a fluid leakage.

In some embodiments, as shown in FIG. 1, FIG. 5 and FIG. 6, the handle assembly 10 further includes a rotation wheel 13, which is sleeved on one end of the tube 31, which end is away from the jaw head assembly 20, wherein the rotation wheel 13 is capable of driving the tube 31 to rotate coaxially. Wherein the rotation wheel 13 is provided with an extension portion 131, and the pull rod 32 extends out of the tube 31, and penetrates through and is arranged at the extension portion 131 and the junction structure 40. The second interface 43 is in seal connection with the extension portion 131, so as to enable the second fluid channel 33 to be connected with the inner cavity 41. In this embodiment, the arrangement of the rotation wheel 13 can facilitate a doctor to adjust an angle of the jaw head assembly 20. At the same time, the extension portion 131 is in sealed connection with the extension portion 131, so as to enable a rotation of the rotation wheel 13 not to affect a fluid to flow in and flow out.

In some embodiments, the junction structure 40 and the handle 11 are integrally formed. In this embodiment, the junction structure 40 can be integrally formed with the handle 11, so as to enable the junction structure 40 to have a stable structure.

Of course, in other embodiments, the junction structure 40 can also be processed separately and mounted at the handle 11. Specifically, the junction structure 40 can be mounted inside the handle 11 and connected with the extension 131 of the rotation wheel 13, by means of a snap connection and/or a threaded connection and/or an interference fit connection, or can be directly placed inside the handle 11 and connected with the extension 131 of the rotation wheel 13, so as to facilitate disassembling the junction structure 40.

For example, the rotation wheel 13 is rotatably provided at the handle 11 and is located at an end of the tube 31, which end is away from the jaw head assembly 20. When the rotation wheel 13 drives the tube 31 to rotate under an external force, the junction structure 40 is fixed in position and does not rotate therewith, so as to facilitate a doctor to adjust an angle of the jaw head assembly 20 without affecting a fluid to flow in and flow out.

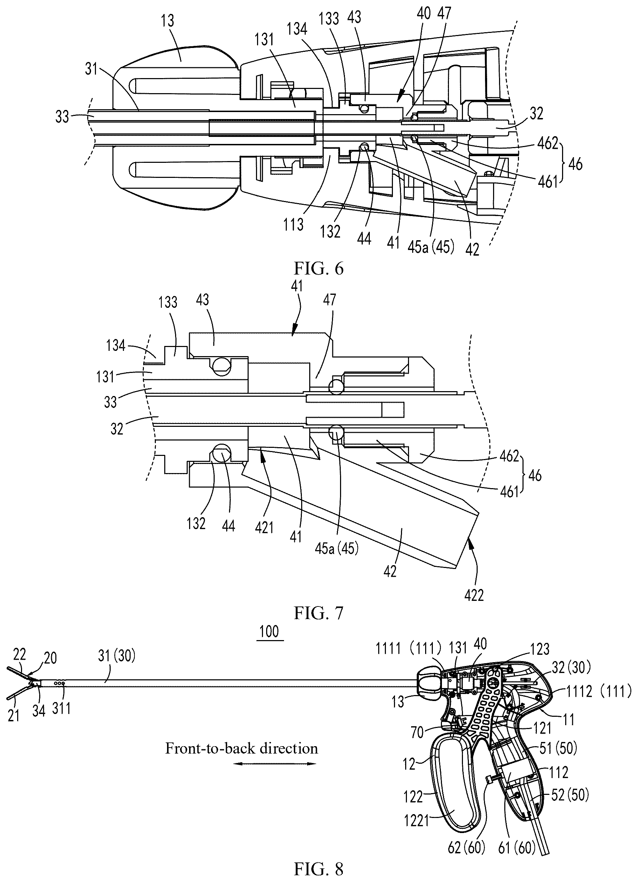

For example, as shown in FIG. 6, a length of the tube 31 extending into the handle 11 can be less than a length of the extension portion 131 extending into the handle 11. In this case, a gap, which is formed between an outer wall of a portion of the pull rod 32 and an inner wall of the extension portion 131, also belongs to a portion of the second fluid channel 33; wherein the portion of the pull rod 32 extends out of the tube 31, penetrates through and is arranged at the extension portion 131. Of course, an end of the tube 31, which end extends into the handle 11, may also be flush with an end of the extension portion 131.

In some embodiments, the second interface 43 elastically abuts against the extension portion 131, so as to maintain the second interface 43 in a sealed fit with the extension portion 131, when the rotation wheel 13 rotates. In this embodiment, the above-mentioned arrangement is adopted, such that on one hand, a sealed fit is maintained between the second interface 43 and the extension portion 131 for keeping airtightness without affecting a rotation of the rotation wheel 13, when the rotation wheel 13 rotates, and on the other hand, a damping effect can be formed due to friction, thereby facilitating an adjustment of an angle of the jaw head assembly 20.

In some embodiments, as shown in FIG. 6 and FIG. 7, one of the second interface 43 and the extension portion 131 is sleeved on the other of the second interface 43 and the extension portion 131, so as to achieve a sealed fit between the second interface 43 and the extension portion 131. In this embodiment, the second interface 43 can be sleeved on the extension portion 131 (as shown in FIG. 7), or the extension portion 131 can be sleeved on the second interface 43, thereby facilitating a sealed connection and maintaining airtightness between second interface 43 and the extension portion 131.

In some embodiments, at least one of the second interface 43 and the extension portion 131 is an elastic structure. In this embodiment, the second interface 43 can be arranged as an elastic structure, or the extension portion 131 can be arranged as an elastic structure, so as to enable the second interface 43 and the extension portion 131 to achieve interference fit through elastic abutment for seal.

It should be noted that, the second interface 43 or the extension portion 131 may also be configured not as an elastic structure, but as a hard structure to improve structural strength and stability. For example, in other embodiments, as shown in FIGS. 6 and 7, the junction structure 40 also includes a first sealing ring 44, and the second interface 43 elastically abuts against the extension portion 131 through the first sealing ring 44. In this embodiment, the second interface 43 is a hard structure, and the first sealing ring can be made of elastic material, so as to achieve elastic abutment between an inner circumferential wall of the second interface 43 and an outer circumferential wall of the extension portion 131 to for seal.

In some embodiments, as shown in FIG. 6 and FIG. 7, the junction structure 40 further includes a sealing member 45. The pull rod 32 penetrates through and is arranged at the junction structure 40, and extends from the sealing member 45 so as to be in a kinematic association with the trigger 12. The sealing member 45 is in a sealed fit with the pull rod 32. In this embodiment, an arrangement of the sealing member 45 can prevent a fluid from leaking from a gap between the junction structure 40 and the pull rod 32, which pull rod 32 penetrates through and is arranged at the junction structure 40.

In some embodiments, the sealing member 45 is an elastic structure, and the junction structure 40 is in a sealed fit with the pull rod 32 through the sealing member 45, which is arranged as an elastic structure, so as to enable the pull rod 32 to maintain airtightness with the junction structure 40, when the pull rod 32 moves along the axial direction of the tube 31. In this embodiment, the sealing member 45 can be integrally formed with the junction structure 40. In this case, the junction structure 40 can entirely be an elastic structure. Of course, the sealing member 45 can also be a separated member which is separately mounted at the junction structure 40. At this time, the junction structure 40 is a hard structure to improve structural strength and stability.

For example, the hard structure mentioned in an embodiment of this disclosure may be made of hard plastic, hard metal or the likes, and the elastic material may be made of silicone, TPE, TPU, PVC, rubber or the likes.

In some embodiments, as shown in FIGS. 6 and 7, when the junction structure is a hard structure, the sealing member 45 can adopt a second sealing ring 45a, and the junction structure 40 also includes a position-limiting member 46. The second sealing ring 45a is sleeved on the pull rod 32 to form a sealed fit between the pull rod 32 and the junction structure 40. The position-limiting member 46 is configured to limit a displacement of the second sealing ring 45a along an axial direction of the tube 31, so as to prevent the second sealing ring 45a from moving with the pull rod 32, when the pull rod 32 moves along the axial direction of the tube 31. In this embodiment, the second sealing ring 45a can be a sealing rubber ring. The arrangement of the second sealing ring 45a can achieve an elastic abutment between the pull rod 32 and the junction structure 40 for seal. Since the pull rod 32 will move along the axial direction of the tube 31, in order to avoid the second sealing ring 45a from moving therewith, a position-limiting member 46 is arranged to prevent the second sealing ring 45a from displacing to affect airtightness.

In some embodiments, as shown in FIGS. 6 and 7, the junction structure 40 is further provided with an abutting portion 47 and a hollow region for accommodating the second sealing ring 45a. The inner cavity 41 is separated from the hollow region by the abutting portion 47. The abutting portion 47 is configured to abut against a side of the second sealing ring 45a, which side faces the inner cavity 41, and the position-limiting member 46 is inserted into the hollow region and abuts against a side of the second sealing ring 45a, which side back-faces the inner cavity 41. In this embodiment, the second sealing ring 45a can be limited between the position-limiting member 46 and the abutting portion 47 by providing the position-limiting member 46 and the abutting portion 47, thereby better preventing the second sealing ring 45a from displacing to affect airtightness.

In some embodiments, as shown in FIGS. 6 and 7, the position-limiting member 46 includes a blocking portion 461, an outer diameter of the blocking portion 461 is adapted to the hollow region of the junction structure 40, and the blocking portion 461 is inserted into the hollow region of the junction structure 40 and abuts against a side of the second sealing ring 45a, which side back-faces the inner cavity 41, meanwhile the blocking portion 461 further abuts against an inner peripheral wall of the hollow region and the outer peripheral wall of the pull rod 32. In this embodiment, the blocking portion 461 is arranged to not only limit the second sealing ring 45a and prevent the second sealing ring 45a from displacing, but also to form a better seal between the junction structure 40 and the pull rod 32, thereby improving a sealing effect.

In some embodiments, as shown in FIG. 6 and FIG. 7, the position-limiting member 46 further includes a covering portion 462, which is connected with the blocking portion 461, wherein the covering portion 462 abuts against a periphery edge of an end of the junction structure 40, which end back-faces the inner cavity. In this embodiment, the covering portion 462 is arranged to facilitate an operation of a doctor, so as to enable the doctor to operate the covering portion 462 to drive the blocking portion 461 to be inserted into or pulled out of the hollow region of the junction structure 40, thereby facilitating disassembly and assembly of the position-limiting member 46 .

In some embodiments, as shown in FIGS. 6 and 7, a first position-limiting ring-groove 132 can be provided at one of the extension portion 131 and the second interface 43 to accommodate the first sealing ring 44, thereby facilitating positioning of the first sealing ring 44. When the rotation wheel 13 rotates, the first sealing ring 44 can also maintain elastic abutment between the extension portion 131 and the second interface 43 without displacing.

In some embodiments, as shown in FIG. 6 and FIG. 7, the outer peripheral wall of the extension portion 131 is further provided with a first position-limiting flange 133. When the second interface 43 is sleeved on the extension portion 131, an end of the second interface 43 abuts against the first position-limiting flange 133 to play a positioning role.

In some embodiments, as shown in FIGS. 6 and 7, a second position-limiting ring-groove 134 can be provided at one of the extension portion 131 and the handle 11, and a second position-limiting flange 113 can be provided at the other of the extension portion 131 and the handle 11. The second position-limiting flange 113 is snap-fitted inside the second position-limiting ring-groove 134, and capable of rotating relative to the second position-limiting ring-groove 134, so as to enable the rotation wheel 13 to drive the extension portion 131 to rotate relative to the main body 111 without displacing in an axial direction.

In some embodiments, as shown in FIG. 6, the pull rod 32 moves along an axial direction of the pull rod 32 that is driven by the trigger, so as to drive the jaw head assembly 20 to open or close, wherein the second interface 43 extends along the axial direction of the pull rod 32, and an angle is formed between an extension direction of the first interface 42 and the axial direction of the pull rod 32. In this embodiment, since the pull rod 32 needs to move along the axial direction, so as to drive the jaw head assembly 20 to open or close, a space needs to be reserved inside the handle 11 along the axial direction of the pull rod 32 for the axial movement of the pull rod 32. The above-mentioned arrangement is adopted, so as to enable the first interface 42 to be tilted to avoid an axial region of the pull rod 32, thereby avoiding interference with the axial movement of the pull rod 32, and reasonably utilizing an idle region inside the handle 11 for obtaining a compact structure, which is conducive to a miniaturization of instrument.

In some embodiments, as shown in FIG. 7, the first interface 42 is provided with a first end 421 which faces the jaw head assembly 20, and a second end 422 which is opposite to the first end 421. The first end 421 is connected with the inner cavity 41. In order to adapt to an internal space of the handle 11, the second end 422 is tilted in a direction away from the pull rod 32 and is connected with the first fluid channel 50 which extends outside the handle 11.

In some usage scenarios, as shown in FIGS. 2, 6-8, the handle 11 is provided with a main body 111 on an upper side 11a and a holding portion 112 on a lower side 11b, along a height direction, the tubing assembly 30 is connected with the main body 111, and the second end 422 is tilted toward the holding portion 112. In this embodiment, the handle 11 is provided with a front side 11c which is adjacent to the jaw head assembly 20, and a rear side 11d which is away from the jaw head assembly 20. The second end 422, that is, an end of the first interface 42, which end is away from the jaw head assembly 20, extends obliquely along a direction away from the holding portion 112 and is connected with the first fluid channel 50. That is, the second end 422 extends toward oblique rear. Without affecting the axial movement of the pull rod 32, this can also facilitate guiding a fluid in a direction toward the holding portion 112. At this time, the first fluid channel 50 can extend out of the holding portion 112 to connect with a fluid delivery device, so as to facilitate guiding a fluid delivery.

Of course, in other embodiments, the first interface 42 may also extend in another direction, as if the first interface 42 is staggered with the pull rod 32.

In some embodiments, the first fluid channel 50 is a hose structure, the fluid delivery device is a negative pressure source, one end of the hose structure is connected with the first interface 42, and the other end of the hose structure extends out of the handle 11 and is connected with the negative pressure source to discharge, through the opening 311, a smoke generated when the jaw head assembly 20 applies energy to a biological tissue. In this embodiment, the fluid path between the opening 311 and the negative pressure source can be used to discharge a smoke generated by an instrument during surgery, so as to prevent the smoke from affecting a surgical field of view of a doctor. The first fluid channel 50 adopts a hose structure, so as to facilitate bending and extending inside the handle 11 to fit an internal space of the handle 11, and the first fluid channel 50 extends out of the handle 11 at a preset position, so as to facilitate discharging smoke generated by the surgical instrument 100.

In some embodiments, as shown in FIG. 3 and FIG. 8-FIG. 10, one end of the first fluid channel 50 is connected with the second fluid channel 33, and the other end of the first fluid channel 50 extends from a bottom of the handle 11 for holding. In this embodiment, one end of the first fluid channel 50 is connected with the second fluid channel 33 at the main body 111, and the other end of the first fluid channel 50 extends from the bottom of the holding portion 112. The junction structure 40 is arranged inside the main body 111, and the first fluid channel 50 extends from the bottom of the holding portion 112, which does not affect an operation of a doctor and makes the handle 11 as a whole beautiful and coordinated.

It should be noted that, a position for the first fluid channel 50 to extend out is not limited to the bottom of the handle 11, and can also be any other position on the handle 11, as long as it does not affect a use of the instrument and an operation of a doctor.

In some embodiments, as shown in FIGS. 8-15, the surgical instrument 100 also includes a switch structure 60, which is capable of switching between a first state and a second state, and acting on a fluid path between the opening 311 and the fluid delivery device. When the switch structure 60 is in the first state, the fluid channel is in an open state, and when the switch structure 60 is in the second state, the fluid channel is in a closed state. In this embodiment, an open/closed state of the fluid channel can be adjusted by arranging the switch structure 60, thereby helping a doctor to quickly control to open and close a fluid delivery. Doctor can control opening the fluid path, when a smoke needs to be discharged or another fluid delivery is needed, and can also control closing the fluid path, when no smoke needs to be discharged or no fluid delivery is needed, so as to reduce energy consumption and save resource.

For example, when performing an operation, such as electrocoagulation or cutting, that is, when the surgical instrument 100 is in an excited state and produces a smoke, a doctor can switch on the switch structure 60 to open the fluid path, thereby discharging a smoke, which is generated when the surgical instrument 100 is excited. When the operation is completed and no smoke is generated, the switch structure 60 can be switched off to cut off the fluid channel.

Optionally, the switch structure 60 may also have a transition state between the first state and the second state, that is, it may gradually switch through the transition state when switching between the first state and the second state. Of course, the switch structure 60 may also have no transition state between the first state and the second state, that is, it may switch directly from the first state to the second state or directly from the second state to the first state.

Optionally, since the first fluid channel 50 is connected with a fluid delivery device, a switch structure 60 may be provided to act on the first fluid channel 50, so as to conveniently control opening or closing the fluid channel by controlling the open/closed state of the first fluid channel 50. Of course, in other embodiments, the switch structure 60 may also act on another position of the fluid channel.

In some embodiments, as shown in FIGS. 8-12, the switch structure 60 is provided at the handle 11. The switch structure 60 includes a valve body 61 and a switch member 62. The valve body 61 is provided at the first fluid channel 50 and is built into the handle 11. When the switch structure 60 is in the second state, the valve body 61 is closed to switch the first fluid channel 50 between an open state and an closed state. One end of the switch member 62 is connected with the valve body 61, and the other end of the switch member 62 extends out of an outer housing of the handle 11. The other end of the switch member 62, which end extends out of the outer housing of the handle 11, is configured to receive a force to drive the switch member 62 to move between a first position and a second position relative to the handle 11. When the switch member 62 moves to the first position, the switch member 62 drives the switch structure 60 to switch to the first state. When the switch member 62 moves to the second position, the switch member 62 drives the switch structure 60 to switch to the second state. In this embodiment, the switch member 62 receives an applied force to drive the valve body 61 to open or close, so as to switch the switch structure 60 between the first state and the second state, thereby facilitating the open-closed adjustment of the first fluid channel 50.

In some embodiments, as shown in FIG. 8-FIG. 9, the handle 11 is provide with a main body 111 and a holding portion 112, the tubing assembly 30 is connected with the main body 111. The trigger 12 includes a connection portion 121 and an operation portion 122. The connection portion 121 is rotatably arranged within the main body 111 and interferes with the pull rod 32 within the main body 111, so as to enable the connection portion 121 to drive the pull rod 32 to move along an axial direction of the tubing assembly 30, when the connection portion 121 rotates. The operation portion 122 is located outside the handle 11 and is arranged opposite to the holding portion 112. The operation portion 122 can be driven by the connection portion 121 to move toward to or away from the holding portion 112. Wherein, one end of the switch member 62, which end extends out of the outer housing of the handle 11, is located on a movement path of the operation portion 122. When the operation portion 122 moves toward the holding portion 112, the operation portion 122 acts on the switch member 62 to drive the switch structure 60 to switch to the first state, so as to enable the first fluid channel 50 to be in an open state. When the operation portion 122 moves away from the holding portion 112, the operation portion 122 acts on the switch member 62 to drive the switch structure 60 to switch to the second state, so as to enable the first fluid channel 50 to be in a closed state. In this embodiment, through the above-mentioned method, the switch structure 60 is constructed to trigger the first fluid channel 50 to open or close, when the trigger 12 moves relative to the handle 11. By linking switching of the switch structure 60 with a movement of the trigger 12, the doctor can control the switch structure 60 while operating the trigger 12 to drive the jaw head assembly 20 to act on a biological tissue, thereby reducing operation steps of the doctor and improving surgical efficiency. The valve body 61 can be opened or closed to adjust an open/closed state of the first fluid channel 50. One end of the switch member 62 is connected with the valve body 61, and the other end of the switch member 62 extends out of the handle 11 and is located on a movement path of the trigger 12. When the trigger 12 moves relative to the handle 11 under an external force, the trigger 12 contacts with the switch member 62 to trigger the switch member 62 to control the valve body 61 to open or close, so as to enable a doctor to control a fluid delivery to open and close more quickly, thereby reducing operation steps of the doctor and improving surgical efficiency.

The connection portion 121 is a portion of an entire trigger 12 that interferes with the pull rod 32, and the operation portion 122 is a portion of an entire trigger 12, which portion extends out of the handle 11 for operation by a doctor. In a specific embodiment, the connection portion 121 and the operation portion 122 are only used to distinguish functions of overall form. In practice, the connection portion 121 and the operation portion 122 may be an integrally formed structure or two separate portions connected with each other. The functional division description of the trigger 12 with respect to the connection portion 121 and the operation portion 122 in the description does not necessarily mean that the connection portion 121 and the operation portion 122 are two separate portions.

For example, the connection portion 121 and the operation portion 122 may be integrally formed, so that a structure of the trigger 12 is more stable and reliable, and is easy to process, thereby reducing a cost of use.

Optionally, as shown in FIGS. 8 and 9, the switch member 62 extends out of a side of the holding portion 112, which side faces the operation portion 122. When the operation portion 122 approaches the holding portion 112, the operation portion 122 can compress the switch member 62, so as to enable the switch member 62 to move to the first position in a direction of being retracted into the handle 11. When the operation portion 122 moves away from the holding portion 112, the operation portion 122 can be separated from the switch member 62. At this time, the switch member 62 can move to the second position to trigger the switch member 62 to control the valve body 61 to open or close, so as to switch the switch structure 60 between the first state and the second state.

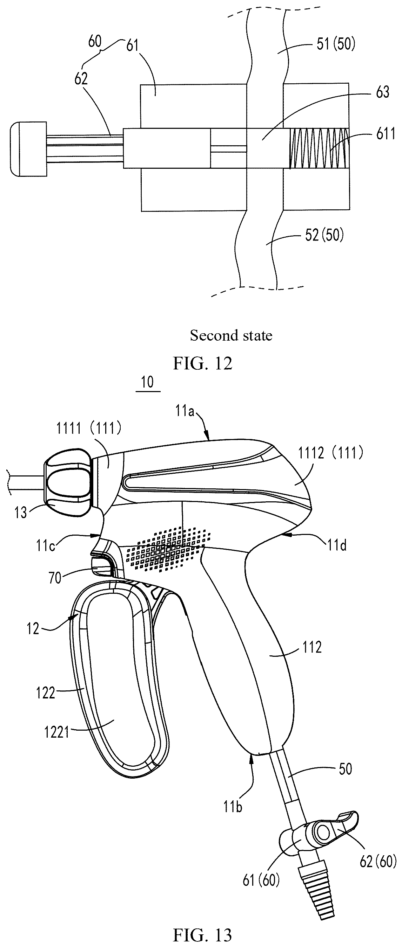

In some embodiments, as shown in FIGS. 11 and 12, when the operation portion 122 compresses the switch member 62 toward the holding portion 112, the valve body 61 is opened by the switch member 62. When the operation portion 122 holding portion 112 is separated from the switch member 62, the switch member 62 can be reset to close the valve body 61.

For example, as shown in FIGS. 8 and 9, when the pull rod 32 moves along a front-to-back direction of the handle 11, and the trigger 12 is located in front of the holding portion 112. The holding portion 112 is used for the doctor to hold. A palm of a doctor holds the holding portion 112, and fingers of the doctor are placed on the operation portion 122 of the trigger 12 to control the operation portion 122, so as to perform an expansion operation and a retraction operation. The retraction operation is to control the operation portion 122 to move toward the holding portion 112, so as to drive the connection portion 121 to rotate backward to drive the pull rod 32 to move backward, in order to close the jaw head assembly 20 to clamp a tissue. At the same time, the operation portion 122 abuts against the switch member 62 to open the valve body 61, so as to open the first fluid channel 50. When fingers of the doctor control the operation portion 122 to perform the expansion operation, that is, to control the operation portion 122 to move away from the holding portion 112, so as to drive the connection portion 121 to rotate forward to drive the pull rod 32 to move forward, in order to open the jaw head assembly 20. At the same time, the operation portion 122 moves away from the switch member 62 to close the valve body 61, so as to close the first fluid channel 50.

Optionally, an elastic member may be provided inside the handle 11 to reset the jaw head assembly 20 to the open state.

As an embodiment, the elastic member can be arranged at an end of the pull rod 32, which end is away from the jaw head assembly 20. At this time, the elastic member can be a compression spring extending along an axial direction of the pull rod 32. When the pull rod 32 moves backward to drive the jaw head assembly 20 to close, the compression spring is compressed. When the doctor releases the operation portion 122 of the trigger 12, an elastic force of the compression spring can act on the pull rod 32, so as to enable the pull rod 32 to move forward to drive the jaw head assembly 20 to reset to the open state.

As another embodiment, as shown in FIGS. 8-10, the elastic member can also be arranged at a hinge point between the connection portion 121 and the main body 111. For example, the connection portion 121 is hinged with the main body 111 through a rotation shaft 123. At this time, the elastic member can adopt a torsion spring arranged on the rotation shaft 123, or the elastic member can be arranged on a movement path of the connection portion 121. When the doctor releases the operation portion 122 of the trigger 12, an elastic force of the torsion spring can act on the connection portion 121, so as to enable the connection portion 121 to drive the pull rod 32 to move forward, in order to drive the jaw head assembly 20 to reset to the open state.

Optionally, a side of the holding portion 112, which side faces the operation portion 122, can be used to limit an extreme position of the operation portion 122, when the operation portion 122 rotates toward the handle 11, so as to enable the jaw head assembly 20 to clamp the tissue under an appropriate clamping force, avoiding unnecessary damage to the tissue due to an excessive clamping force. Of course, other blocking structures may also be provided in the holding portion 112 to limit the extreme position of the operation portion 122 or the connection portion 121, when the operation portion 122 rotates toward the handle 11 or when the connection portion 121 moves toward the handle 11.

Of course, in other embodiments, it may be configured that, when the operation portion 122 compresses the switch member 62 toward the holding portion 112, the valve body 61 can be closed by the switch member 62, and when the operation portion 122 and the holding portion 112 are separated from the switch member 62, the valve body 61 can be opened by the switch member 62.

Alternatively, in other embodiments, it may be configured that, when the operation portion 122 compresses the switch member 62 for a first time, the valve body 61 is opened by the switch member 62, and when the operation portion 122 compresses the switch member 62 again, the valve body 61 is closed by the switch member 62.

As an embodiment, as shown in FIGS. 11 and 12, a portion of the valve body 61, which portion acts on the first fluid channel 50, can adopt a switch valve provided with a valve 63. The first fluid channel 50 is opened and closed by driving the valve 63 to block or open a preset position of the first fluid channel 50. At this time, the switch element 62 serves as a portion that receives a force. A force applied from outside can act on the switch element 62 to switch the switch structure 60 between the first state and the second state, thereby driving the valve 63 to block or open the preset position of the first fluid channel 50.

For example, as shown in FIGS. 8, 9, 11 and 12, the first fluid channel 50 is divided into a first section 51 and a second section 52 by arranging the valve body 61. The first section 51 is connected with the second fluid channel 33 through the junction structure 40, and the second section 52 extends out of the handle 11 and is connected with the fluid delivery device. The valve body 61 can have two interfaces, and the two interfaces of the valve body 61 are respectively connected with the first section 51 and the second section 52. One end of the switch member 62 is connected with the valve 63 inside the valve body 61 and can drive the valve 63 to move inside the valve body 61. The other end of the switch member 62 extends out of the handle 11 and is located on the movement path of the trigger 12. When the switch member 62 is in an initial state, the valve body 61 is closed. When the operation portion 122 of the trigger 12 moves adjacent to the holding portion 112 of the handle 11, the operation portion 122 compresses an end of the switch member 62 which end extends out, so as to push an end of the switch member 62 which end locates inside the valve body 61 to move to a first position, thereby driving the valve 63 to move for opening the valve body 61, so as to further open the first fluid channel 50. A reset member 611, such as a spring, can be provided inside the valve body 61. When the operation portion 122 of the trigger 12 moves to separate from the switch member 62, the reset member 611 can provide an elastic force to push the switch member 62 to a second position, so as to enable the switch member 62 to drive the valve 63 to reset for closing the valve body 61, so as to further close the first fluid channel 50.

As another embodiment, the valve body 61 is a portion that acts on the first fluid channel 50, and can be a clamping structure, which is capable of opening or closing the first fluid channel 50 by clamping or loosening the first fluid channel 50. At this time, the switch element 62 serves as a portion that receives a force. A force applied from outside can act on the switch element 62 to drive the valve body to open or close, so as to clamp or loosen the first fluid channel 50.

Wherein, the valve body 61 is a portion of the entire switch structure 60, which portion acts on a fluid path, and the switch member 62 is a force-bearing unit. In a specific embodiment, the valve body 61 and the switch member 62 are only used to distinguish functions of overall form. In fact, the valve body 61 and the switch member 62 can be an integrally formed structure or two separately connected members. The functional division description of the valve body 61 and the switch member 62 of the switch structure 60 in the description does not necessarily mean that the valve body 61 and the switch member 62 are two separate members. The description of the valve body 61 and the switch member 62 in the description is only used to explain the switch structure 20, the fluid path and the force conditions.

For example, as shown in FIGS. 2, 3 and 10, the operation portion 122 of the trigger 12 can be in a closed loop state, with an inner hole 1221 for a finger to be inserted into, and an inner wall of a front side 11c of the inner hole 1221 and an inner wall of a rear side 11d of the inner hole 1221 can be provided with an arc structure that fits the finger, so as to enable a finger of a doctor to abut against, thereby facilitating an expansion or retraction operation of the operation portion 122.

It should be noted that the switch structure 60 mounted at the handle 11 may also have no kinematic fit with the trigger 12. For example, in other embodiments, as shown in FIG. 10, the handle 11 is provided with a main body 111 and a holding portion 112, and the tubing assembly 30 is connected with the main body 111. The trigger 12 includes a connection portion 121 and an operation portion 122. The connection portion 121 and the operation portion 122 can be integrally formed or separately connected. The connection portion 121 can be rotatably arranged inside the main body 111 and interfere with the pull rod 32 inside the main body 111, so as to drive the pull rod 32 to move along an axial direction of the tubing assembly 30, when the connection portion 121 rotates. The operation portion 122 is located outside the handle 11 and is arranged opposite to the holding portion 112. The operation portion 122 can be driven by the connection portion 121 to move toward or away from the holding portion 112. One end of the switch member 62, which end extends out of an outer housing of the handle 11, is located outside a movement path of the operation portion 122. In this embodiment, the switch member 62 may also extend from any position of the outer housing of the handle 11, which position is outside the movement path of the operation portion 122, as long as the switch member 62 does not affect a use of the instrument and an operation of a doctor.

In some embodiments, as shown in FIG. 10, one end of the switch member 62 is connected with the valve body 61, and the other end of the switch member 62 extends out of a side of the handle 11, which side back-faces the trigger 12. The switch member 62 controls the valve body 61 to close and open under an applied external force. In this embodiment, the switch member 62 may also be arranged on a side of the handle 11, which side back-faces the trigger 12 , so as not to interfere with an operation of the trigger 12.

In some embodiments, as shown in FIG. 10, the handle 11 is provided with a front side 11c which faces the jaw head assembly 20, and a rear side 11d which back-faces the jaw head assembly 20, along an extension direction of the pull rod 32, the trigger 12 is located on the front side 11c of the handle 11, and the switch member 62 extends from the rear side 11d of the handle 11. In this embodiment, the trigger 12 is arranged on the front side 11c of the handle 11, which is convenient for the doctor to expand and retract the trigger 12. The switch member 62 is arranged on the rear side 11d of the handle 11, so as not to interfere with the operation of the trigger 12 and not affect an operation of a doctor of the surgical instrument 100. When the valve body 61 needs to be opened, the switch member 62 is pressed to deliver a fluid to be discharged.

In some embodiments, as shown in FIG. 10, the handle 11 is provided with a main body 111 on an upper side 11a, and a holding portion 112 on a lower side 11b, along a height direction. The tubing assembly 30 is connected with the main body 111, and the trigger 12 is located on the front side 11c of the holding portion 112. The main body 111 is provided with a head portion 1111 located at the front side 11c, and a tail portion 1112 located at the rear side 11d, wherein the switch member 62 extends out of the tail portion 1112. In this embodiment, the above arrangement not only avoids affecting holding of a doctor on the holding portion 112 and an operation of the trigger 12 of the doctor, but also makes it convenient for the doctor to press the switch member 62 from the tail portion 1112.

For example, as shown in FIG. 5, in order to reduce a volume of the handle 11, an end of the pull rod 32 is adjacent to an inner wall of the tail portion 1112. At this time, a preset space is provided between the end of the pull rod 32 and the inner wall of the tail portion 1112, so as to enable the pull rod 32 to move backward for driving the jaw head assembly 20 to close. The switch structure 60 and the pull rod 32 are staggered to avoid interfering with a movement of the pull rod 32.

It should be noted that, the switch structure 60 is not limited to being mounted at the handle 11. For example, in other embodiments, as shown in FIG. 13, one end of the first fluid channel 50 is connected with the second fluid channel 33, and the other end of the first fluid channel 50 extends out of the outer housing of the handle 11. The switch structure 60 is provided at the first fluid channel 50, which extends out from an outer housing of the handle 11. In this embodiment, the switch structure 60 can be independently provided at a portion of the first fluid channel 50, which portion extends outside of the handle 11, that is, located outside the handle 11, so as to adjust an open/closed state of the first fluid channel 50. The switch structure 60 is not in contact with the handle 11 and is far away from an operation position of doctor, which can better avoid affecting an operation of a doctor of the surgical instrument 100.

Optionally, as shown in FIGS. 13-15, the switch structure 60 includes a valve body 61 and a switch member 62. The valve body 61 is arranged at the first fluid channel 50 which extends out of the outer housing of the handle 11. The valve body 61 can be opened or closed to adjust an open/closed state of the first fluid channel 50. The switch member 62 is connected with the valve body 61, and is configured to receive a force to drive the switch structure 60 to switch between the first state and the second state. In this embodiment, the switch member 62 is constructed to be able to control the valve body 61 to open or close under an external force. The switch structure 60 is placed outside the handle 11, which facilitates assembly, disassembly and maintenance of the switch structure 60, and the switch structure 60 can be kept away from the handle 11, thereby better avoiding affecting an operation of a doctor of the surgical instrument 100.

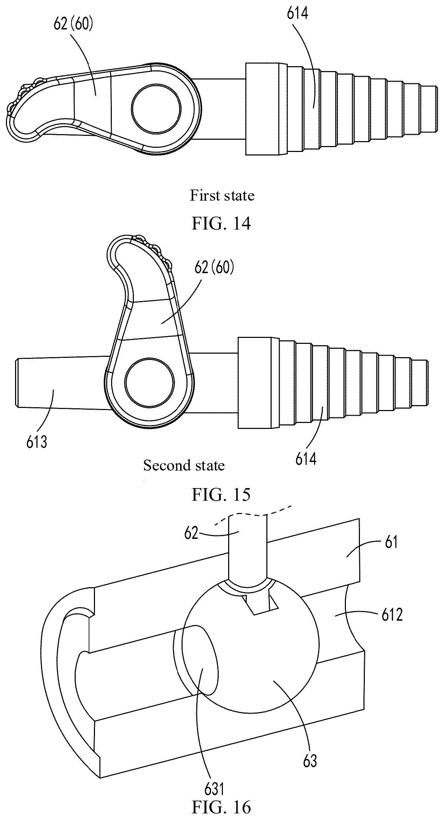

In some embodiments, as shown in FIG. 16, the valve body 61 is provided with a connection channel 612 which is connected with the first fluid channel 50, and a valve 63 which is movably arranged inside the connection channel 612. The switch element 62 is connected with the valve 63, and is configured to receive a force to drive the valve 63 to move, so as to open or close the connection channel 612, thereby switching the first fluid channel 50 between an open state and a closed state. In this embodiment, the valve body 61 is further provided with a first outlet 613 and a second outlet 614, that are connected with the connection channel 612. The first outlet 613 is connected with the first fluid channel 50, and the second outlet 614 is connected with the fluid delivery device. The valve 63 is movably arranged inside the connection channel 612. The switch member 62 is connected with the valve body 61 through the valve 63. The switch member 62 can drive the valve 63 to move under an external force, so as to adjust the valve 63 to open or close the connection channel 612, thereby controlling the valve body 61 to open and close. Through the above arrangement, the opening or closing of the connection channel 612 can be conveniently controlled to adjust an open-closed state of the first fluid channel 50.

Optionally, as shown in FIG. 16, the valve 63 can be provided with a penetrated through hole 631, and the valve 63 can be rotatably arranged inside the connection channel 612. The switch member 62 is configured to receive a force to drive the valve 63 to rotate, that is, the switch member 62 can drive the valve 63 to rotate under an external force, so as to adjust an angle between a penetration direction of the through hole 631 and a penetration direction of the connection channel 612, thereby adjusting a size of a region of the connection channel 612 for a fluid to flow through. In this embodiment, the above arrangement can be used to adjust the through hole 631 and the connection channel 612 to be opposite or offset to each other. By adjusting an offset angle between the through hole 631 and the connection channel 612, a flow rate for a fluid, that can flow through the connection channel 612, can be adjusted. For example, the valve body 61 may adopt a spherical structure or a hemispherical structure of the through hole 631 to facilitate rotation.

Optionally, when a penetration direction of the through hole 631 is parallel to a penetration direction of the connection channel 612, the switch member 62 rotates to the first position and the connection channel 612 is completely opened. When the penetration direction of the through hole 631 is perpendicular to the penetration direction of the connection channel 612, the switch member 62 rotates to the second position and the connection channel 612 is closed. When the switch member 62 rotates from the first position to the second position, a region of the connection channel 612 through which the fluid flows, gradually decreases. In this embodiment, when the penetration direction of the through hole 631 is parallel to the penetration direction of the connection channel 612, the through hole 631 is opposite the connection channel 612, and the connection channel 612 is completely opened, so as to enable a flow rate of a fluid which flows through the connection channel 612 to reach a maximum. When the penetration direction of the through hole 631 is perpendicular to the penetration direction of the connection channel 612, the through hole 631 and the connection channel 612 are completely staggered, so as to prevent the fluid from flowing through the connection channel 612, that is, the connection channel 612 is closed. Through the above arrangement, the connection channel 612 can be adjusted between completely opened, partially opened and closed states, so as to satisfy a use requirement of a doctor.

Optionally, the switch structure 60, which is provided at the first fluid channel 50 outside the handle 11, is not limited to embodiments shown in FIGS. 14 to 16, but may also adopt embodiments shown in FIGS. 11 and 12, or other embodiments that can control the first fluid channel 50 to open and close.

Optionally, the switch structure 60, which is arranged at the handle 11, is not limited to embodiments shown in FIGS. 11 and 12, and can also adopt embodiments shown in FIGS. 14 to 16, or other embodiments that can control the first fluid channel 50 to open and close.

It should be noted that, in addition to being able to adjust the first fluid channel 50 to open and close, through an external force, that is, through a physical contact to control the switch structure 60, the switch structure 60 can also be controlled by a computer program, that is, through existing software to control the switch structure 60 to close and open.

For example, in some further embodiments, the surgical instrument 100 further includes a control module for outputting an electrical signal to the switch structure 60 to drive the switch structure 60 to switch between the first state and the second state. In this embodiment, the switch structure 60 can be arranged as an electromagnet switch, so as to enable the control module to output an electrical signal to the electromagnet switch for controlling the switch structure 60 to open and close, and thus further controlling the first fluid channel 50 to open and close. The control module can be controlled according to existing computer programs without requiring manual operation, thereby improving a surgical efficiency of doctor.

As an embodiment, the control module includes a monitoring unit and a control unit. The monitoring unit is configured to output a signal when detecting the surgical instrument 100 to be in an excited state, and to control the jaw head assembly 20 to apply energy to a biological tissue when the surgical instrument 100 is in the excited state. The control unit is configured to receive the outputted signal of the monitoring unit and output an electrical signal to the switch structure 60 to drive the switch structure 60 to switch between the first state and the second state, thereby controlling the first fluid channel 50 to open and close. In this embodiment, the control unit is configured to control the switch structure 60 to open and close, and the monitoring unit is configured to monitor whether the surgical instrument 100 is in an excited state. When the surgical instrument 100 is in an excited state, a smoke will be generated. The monitoring unit sends a signal to the control unit, and the control unit controls the switch structure 60 to open, so that the first fluid channel 50 is open to discharge the smoke.

In some embodiments, as shown in FIGS. 1 and 2, the surgical instrument 100 also includes a function button 70, which can be arranged on the front side 11c of the handle 11, so as to facilitate the doctor to trigger the function button 70 with his index finger when holding the handle 11, in order to control the jaw head assembly 20 to apply energy to a tissue in a tissue clamped region, and enable the surgical instrument 100 to be in an excited state.

The embodiment of this disclosure further provides a surgical system, including a surgical energy host and the above-mentioned surgical instrument 100. The surgical energy host is connected with the surgical instrument 100. In this embodiment, the surgical energy host can provide energy to the surgical instrument 100, can also be configured to provide an electrical signal to the surgical instrument 100, and can also be configured to receive a signal fed back from the surgical object and perform a relevant analysis. A structure and function of the surgical instrument 100 in the surgical system proposed in an embodiment of this disclosure are the same as those in the above embodiment. Please refer to the description of the above embodiment for details, and this embodiment will not be repeated.

Those skilled in the art may integrate and combine different embodiments or examples described in the description, and features of the different embodiments or examples without mutual contradiction.

The above description is only a specific implementation of this disclosure, a scope of protection of this disclosure is not limited thereto. Any technician familiar with the technical field can easily think of various equivalent modifications or replacements within the technical scope disclosed in this disclosure, and these modifications or replacements should be included in the scope of protection of this disclosure. Therefore, the protection scope of this disclosure should be based on the protection scope of the claims.

## Claims

1. A surgical instrument, **characterized in that**, comprising:
a handle assembly, which comprises a handle for holding and a trigger capable of moving relative to the handle, wherein the handle is provided with a first fluid channel;
a jaw head assembly, which comprises a first jaw head and a second jaw head, wherein the first jaw head and the second jaw head are capable of pivoting relative to each other, so as to open or close the jaw head assembly, wherein at least one of the first jaw head and the second jaw head is configured to implement a treatment on a biological tissue;
a tubing assembly, which comprises a pull rod and a tube which is sleeved outside the pull rod, wherein the tubing assembly is configured to connect the jaw head assembly and the handle; when the trigger moves relative to the handle, the jaw head assembly moves under an action of the pull rod, which pull rod is in a kinematic fit with the trigger, so as to enable the first jaw head and the second jaw head to pivot relative to each other;
wherein, a second fluid channel is formed between an outer peripheral wall of the pull rod and an inner peripheral wall of the tube; a position of the tube, which position is adjacent to the jaw head assembly, is provided with an opening which is connected with the second fluid channel; wherein the first fluid channel is connected with the second fluid channel and is further connected with a fluid delivery device, so as to form a fluid path between the opening and the fluid delivery device.

2. The surgical instrument according to claim 1, **characterized in that**, further comprising:
a junction structure, which is configured to seal and connect the first fluid channel and the second fluid channel, so as to form the fluid path between the opening and the fluid delivery device.

3. The surgical instrument according to claim 2, **characterized in that**, the junction structure comprises an inner cavity, and a first interface and a second interface which are connected with the inner cavity; wherein the first interface is configured to seal and connect the inner cavity and the first fluid channel, and the second interface is configured to seal and connect the inner cavity and the second fluid channel.

4. The surgical instrument according to claim 3, **characterized in that**, the handle assembly further comprises a rotation wheel, which is sleeved on an end of the tube, which end is away from the jaw head assembly, wherein the rotation wheel is capable of driving the tube to rotate coaxially;
wherein the rotation wheel is provided with an extension portion, the pull rod extends out of the tube, and then penetrates through and is arranged at the extension portion and the junction structure, wherein the second interface is in sealed connection with the extension portion, so as to connect the second fluid channel with the inner cavity.

5. The surgical instrument according to claim 4, **characterized in that**, the second interface elastically abuts against the extension portion, so as to maintain a sealed fit between the second interface and the extension portion, when the rotation wheel rotates.

6. The surgical instrument according to claim 4, **characterized in that**, one of the second interface and the extension portion is sleeved on the other one of the second interface and the extension portion, so as to achieve a sealed fit between the second interface and the extension portion.

7. The surgical instrument according to claim 4, **characterized in that**, at least one of the second interface and the extension portion is an elastic structure, or
the junction structure further comprises a first sealing ring, wherein the second interface elastically abuts against the extension portion through the first sealing ring.

8. The surgical instrument according to claim 2, **characterized in that**, the junction structure further comprises a sealing member, wherein the pull rod penetrates through and is arranged at the junction structure, and extends from the sealing member so as to be in a kinematic association with the trigger, wherein the sealing member is in a sealed fit with the pull rod.

9. The surgical instrument according to claim 8, **characterized in that**, the sealing member is an elastic structure, and the junction structure is in a sealed fit with the pull rod through the elastic structure, so as to maintain airtightness between the pull rod and the junction structure, when the pull rod moves along an axial direction of the tube.

10. The surgical instrument according to claim 3, **characterized in that**, the junction structure further comprises a second sealing ring and a position-limiting member, wherein the second sealing ring is sleeved on the pull rod, so as to form a sealed fit between the pull rod and the junction structure, the position-limiting member is configured to limit a displacement of the second sealing ring along an axial direction of the tube, so as to prevent the second sealing ring from moving with the pull rod, when the pull rod moves along the axial direction of the tube.

11. The surgical instrument according to claim 10, **characterized in that**, the junction structure is further provided with an abutting portion and a hollow region which is configured to accommodate the second sealing ring, the inner cavity is separated from the hollow region via the abutting portion, the abutting portion abuts against a side of the second sealing ring, which side faces the inner cavity, the position-limiting member is inserted into the hollow region and abuts against a side of the second sealing ring, which side back-faces the inner cavity.

12. The surgical instrument according to claim 3, **characterized in that**, the pull rod moves along an axial direction of the pull rod that is driven by the trigger, so as to drive the jaw head assembly to open or close;
the second interface extends along the axial direction of the pull rod, and an angle forms between an extension direction of the first interface and the axial direction of the pull rod.

13. The surgical instrument according to claim 12, **characterized in that**, the first interface is provided with a first end which faces the jaw head assembly, and a second end which is opposite to the first end; wherein the first end is connected with the inner cavity, the second end is tilted in a direction away from the pull rod and is connected with the first fluid channel which extends outside the handle.

14. The surgical instrument according to claim 3, **characterized in that**, the first fluid channel is a hose structure, the fluid delivery device is a negative pressure source, one end of the hose structure is connected with the first interface, the other end of the hose structure extends out of the handle and is connected with the negative pressure source, so as to discharge, through the opening, a smoke which is generated when the jaw head assembly applies energy to the biological tissue.

15. The surgical instrument according to claim 1, **characterized in that**, one end of the first fluid channel is connected with the second fluid channel, the other end of the first fluid channel extends from a bottom of the handle.

16. The surgical instrument according to claim 2, **characterized in that**, the junction structure is integrally connected with the handle; or
the junction structure is in snap-fit connection, thread connection or interference-fit connection with the handle.

17. The surgical instrument according to claim 1, **characterized in that**, further comprising:
a switch structure, which is configured to switch between a first state and a second state, and act on the first fluid channel; wherein the first fluid channel is in an open state, when the switch structure is in the first state; the first fluid channel is in a closed state, when the switch structure is in the second state.

18. The surgical instrument according to claim 1, **characterized in that**, the tube is affixed with an insulation layer at least at the opening, so as to prevent an electrical leakage at the opening while the surgical instrument is in use.

19. The surgical instrument according to claim 18, **characterized in that**, the tube comprises a front section which is adjacent to the jaw head assembly, wherein the opening is arranged at the front section, and the insulation layer is affixed to an outer peripheral wall of the front section and an inner peripheral wall of the front section, which inner peripheral wall is adjacent to the opening.

20. The surgical instrument according to claim 1, **characterized in that**, in order to enable at least one of the first jaw head and the second jaw head to apply energy from a surgical energy host to the biological tissue:
the tube is in electrical connection with the first jaw head, the pull rod is in electrical connection with the second jaw head, wherein the tube and the pull rod are relatively insulated with each other; or
the pull rod comprises a first conductive unit and a second conductive unit that are independent of each other, wherein the first conductive unit is in electrical connection with the first jaw head, and the second conductive unit is in electrical connection with the second jaw head; or
the tube is insulated from the pull rod, and the surgical instrument further comprises a first wire and a second wire, wherein the first wire is in electrical connection with the first jaw head, and the second wire is in electrical connection with the second jaw head.

21. A surgical system, **characterized in that**, comprising:
the surgical instrument according to claim 1;
a surgical energy host, which is connected with the surgical instrument to provide energy to the surgical instrument.
